Europäisches Patentamt

European Patent Office

Office européen des brevets

(19)

(11) Publication number: 0 355 910
A1

## EUROPEAN PATENT APPLICATION

(21) Application number: 89202076.9

(51) Int. Cl.⁴: C12M 1/12

(22) Date of filing: 11.08.89

(30) Priority: 18.08.88 BE 8800941

(43) Date of publication of application:
28.02.90 Bulletin 90/09

(84) Designated Contracting States:
AT CH DE ES FR GB GR IT LI LU NL SE

(71) Applicant: SCHELDE-DELTA, b.v.b.a.
Arenbergstraat 23
B-2000 Antwerpen(BE)

(72) Inventor: Martens, Laurent
Gustaaf Papestraat 74
B-9300 Aalst(BE)

(74) Representative: Donné, Eddy
M.F.J.Bockstael Arenbergstraat 13
B-2000 Anvers(BE)

(54) Reactor and procedure for realising a fermentation process which such a reactor utilises.

(57) Reactor, for the production of a bioconversion or chemical reaction of the type whereby a product to be treated is transformed by means of an auxiliary medium into a product of which the overall density is different from the density of the product to be treated, characterised in that the reactor principally consists of means (2) which can provide for the immobilisation of an auxiliary medium (3) dispersed in space, consisting of a porous structure (10); an inlet (4) for the supply of the product (5) to be treated; at least one free channel (6), extending upwards connected with one extremity to this inlet (4) that allows that the product (5) to be treated makes contact along the walls (7) of this with the immobilised auxiliary medium (3); and an outlet (8) provided on the other extremity of the channel (6).

Fig.1

## Reactor and procedure for realising a fermentation process which such a reactor utilises.

This invention relates to a reactor for the production of a bioconversion or a chemical reaction, or similar transformation.

Generally it concerns a reactor which is destined for the production of bioconversions or chemical reactions of the type whereby a product to be treated by means of an accelerating medium such as an organism, a catalyst or similar is transformed into a product of which the overall density is different from the density of the product to be treated.

In particular the invention concerns a reactor which permits that a fermentation process can be realized without the microorganisms required for this freely arriving in the nutrient medium. The reactor is therefore very suitable to be applied in a favourable manner in the production of beers and other alcoholic beverages.

The invention as a result also relates to a procedure for realising a fermentation process, in particular for realising an alcoholic yeasting.

From the European patent application no. 112.812 a biocatalytic reactor is known, which consists in a first zone and a second zone, which are separated from each other by means of a semipermeable membrane, for which purpose use is made of hollow fibre technique. In use the semipermeable membrane is penetrable for the product to be reacted as well as for the formed end product, while it is impenetrable for the microbiological cell material applied as catalyst. The material to be reacted is added to the first zone, penetrates the semipermeable membrane and in so doing comes into contact with the catalyst present in the second zone, after which the formed end product can penetrate back into the first zone, after which this product of the material to be reacted can be separated. The use of such semipermeable membrane shows the disadvantage that the dead and disintegrated organisms remains present in the second zone, so that after some time this zone becomes blocked and the growth of new organisms is hindered. As a result the reactor needs to be shut down regularly to rinse through the second zone by means of a dissolvent.

Also from the US 4.266.026 the use of such biocatalytic, reactor is known, whereby as in the aforementioned European patent application use is made of hollow fibres. The use of such hollow fibres shows the disadvantage that, in view of the very small diameter of the through channel, they hardly allow the passage of gas bubbles, unless extremely powerful pumps are used.

The present invention relates to a reactor which does not show the aforementioned disadvantages, more especially whereby, on the one hand, the remaining product of dead and disintegrated organism does not impede the proper operation of the reactor, and on the other hand, a good flow is possible in the reactor, whereby an automatic flow is provided without a pump being required for this.

The cleaning of the dirty pump is therefore no longer necessary.

The reactor according to the invention shows the characteristic that it principally consists of means which can provide for the immobilisation, dispersed in space of an auxiliary medium, such as an organism, a catalyst or similar; an inlet for the supply of the product to be treated; at least one free channel extending upwards connected with one extremity to this inlet, that allows that the product to be treated makes contact along the walls of this with the immobilised auxiliary medium; and an outlet provided on the other extremity of the channel.

In the preferred embodiment the outlet and the inlet of the reactor are connected to each other by means of a free feed back pipe, such that an automatic circulation develops as long as the reaction or bioconversion manifests itself, such through the appearing overall density difference that develops in the channel of the reactor. In the case of an alcoholic yeasting such embodiments offers the advantage that no circulation pumps are required so that these cannot form an origin of infection.

The means which can provide for an immobilised catalyst, organism or similar, consist of a porous structure through which the aforementioned channel of the reactor extends, whereby this structure is so conceived that it allows a catalyst of microorganism to be rinsed in without this arriving in the channel of the reactor. A procedure as described in the Belgian patent application no. 8700850 of applicant is preferably utilised for immobilising the catalyst or the organisms.

In order to show better the characteristics according to the present invention, some preferred embodiments are described hereafter, as examples and without any restrictive character, with reference to the enclosed drawings, in which:

figure 1 shows a laboratory arrangement of a reactor according to the invention, in particular for realising a fermentation process;

figure 2 shows on a larger scale a view of the part that is intended by F2 in figure 1, in particular while the microorganisms, which are necessary for the yeasting process, are being rinsed in;

figure 3 shows a variant of the part that is illustrated in figure2;

figures 4 and 5 show for various positions the part that is indicated by F4 in figure 1;

figure 6 shows in cross-section a variant for the reactor;

figure 7 shows very schematically a practical embodiment of the device according to figure 1;

figure 8 shows on a larger scale a cross-section of the part that is indicated by F8 in figure 6.

In figure a reactor 1 according to the invention is shown, in particular for realising a fermentation process. The actual reactor fundamentally consists hereby of means 2 which can provide for an immobilised auxiliary medium such as a catalyst, organisms or similar - which are schematically indicated by means of crosses 3; an inlet 4 for the supply of the product 5 to be treated; at least one free channel 6 extending upwards connected with one extremity to this inlet 4, that allows that the product 5 to be treated makes contact along the walls 7 of this with the catalyst or the organisms 3 and an outlet 8 provided on the other extremity of the channel. The channel 6 - or possible several channels 6 - is preferably perpendicular.

The outlet 8 and the inlet 4 are outside the real core of the reactor 1 preferably connected to each other by means of a feed back pipe 9, such that by the channel 6 and the feed back pipe 9 a circuit with a free flow opening is formed.

The means 2 which are to be provided for an immobilised catalyst or organism, in other words a catalyst or organism that cannot be carried along with the Product 5, consist of a porous structure 10 through which the aforementioned channel 6 extends, whereby this structure 10 is formed by one or several materials which allow an auxiliary medium 3 in solid state to be rinsed in. For this purpose the means 2 include a cover 11 which surrounds the outside of the porous structure 10, whereby a space 12 is left between this cover 11 and the structure 10. The cover 11 has at least one supply opening 13 which is provided with a cutoff 14, the purpose of which is explained later.

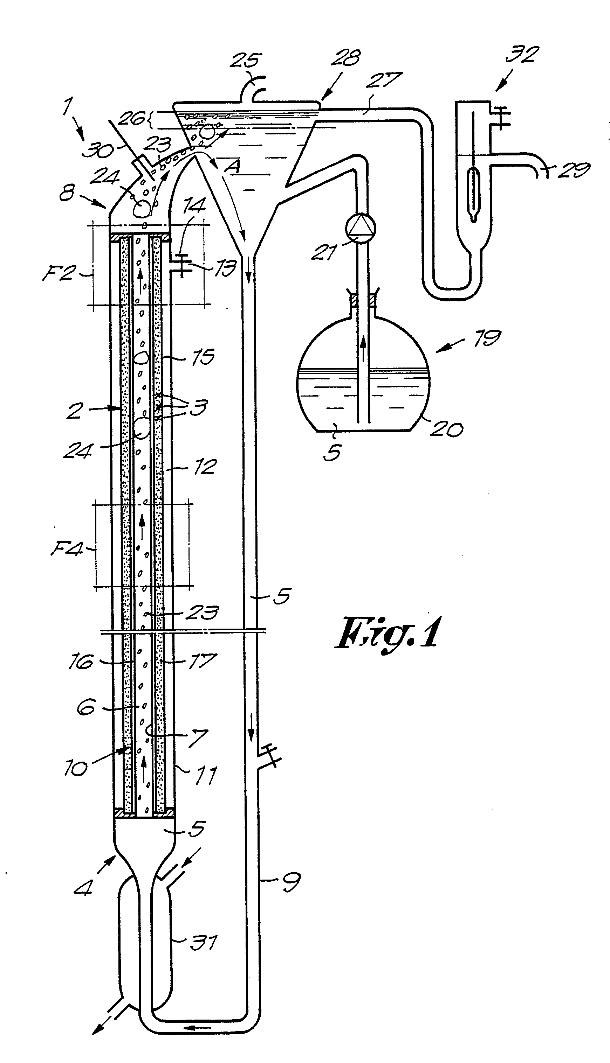

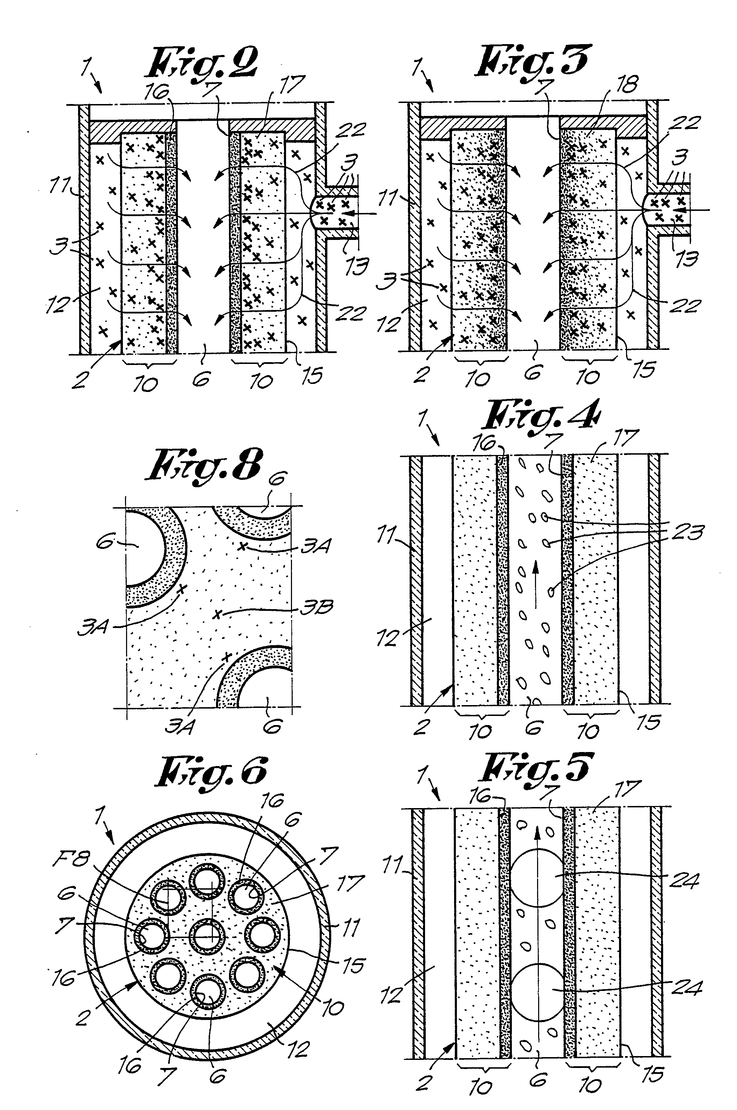

In order now to achieve that catalyst particles or organisms may be immobilised in the porous structure 10, use is made of a structure of which the degree of fineness of the porosity increases in the direction of the channel 6, or respectively the channels 6, such that by rinsing in the desired catalyst or organisms 3 via the supply opening 13 and the space 12 in the structure 10, these can press along the outer wall 15 into the structure 10 considering the relative coarse porosity, but cannot leave this structure considering the fine porosity of this near the wall 7. According to figure 2 use is made of a porous structure 10 for this purpose which principally consists of two layers 16 and 17 fitting closely to each other as a physical entity.

The material of the layer 16 which borders on the channel 7, shows the finest porosity, more especially a porosity whereby the catalyst 3 concerned or the organisms concerned which will be applied in the reactor cannot penetrate through this layer 16. The layer 17 has a less fine porosity, all of which such that this can act as a holder for the catalyst concerned or the organism 3 concerned.

The layer 16 preferably consists of aluminium oxide, while the layer 17 is formed by silicon carbide. It is however clear that other materials can also be applied. So can the layer 16 also consist of silicon oxide for example, while the layer 17 can be formed of boron carbide, aluminosilicate, and suchlike.

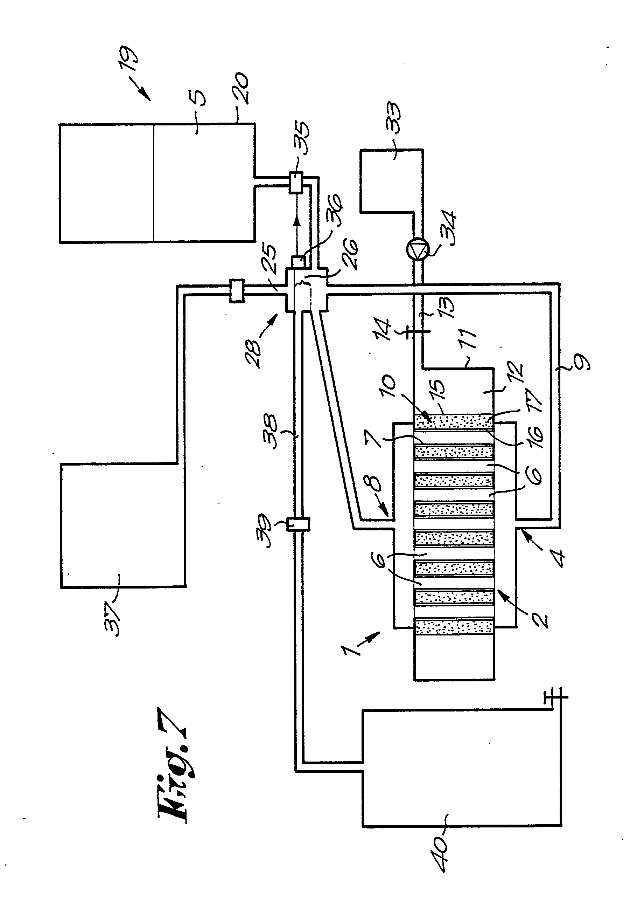

According to the variant which is shown in figure 3, the porous structure 10 principally consists of one layer 18 acting as a holder whereby the fineness of the porosity gradually increases in the direction towards the channel 6. The variable porosity is then chosen in function of the size of the catalyst or organisms to be immobilised, obviously again such that the porosity on the outer wall 15 allows the penetration of the catalyst or organisms 3, while through the gradual increase of the porosity this catalyst or organisms 3 come to be stuck in a well defined place in the structure 10. Such layer 18 can for example consist of silicon carbide.

The circuit of the reactor according to figure 1 is preferably connected to supply means 19 which can, either once per reaction cycle, or continuously, provide for the supply of the product 5 to be treated. These supply means 19 can consist of a reservoir 20 that contains the product 5 and a, preferably controlled, pumping device 21 to supply the product 5 to the circuit. According to a variant the reservoir 20 can also be placed above the circuit, such that a pumping device 21 is superfluous and can be replaced by a controlled regulating valve.

The operation of the reactor according to the invention is now described hereafter on the basis of figures 1, 2, 4 and 5, in particular in a utilisation of it whereby a fermentation process is realised.

A first step hereby consists in that the porous structure 10 is loaded with organisms 3, for example Zymomonas Mobylis, which by means of a stream of liquid 22 is rinsed along the supply opening 13 and the space 12 into the structure 10. The living organisms 3 come to be dispersed through this in the layer 17 and are absolutely prevented from penetrating into the channel 6 by the presence of the fine layer 16. Subsequently the cutoff 14 is closed and the organisms 3 can through their growth further develop in the layer 17.

The circuit formed by the channel 6 of the reactor and the pipe 9 is filled with the product 5 to be treated. In order to provide for the production of alcoholic beverages, a so-called nutrient medium is used for this, that as known for example consists of wort or a replacement synthetic medium in the case of the production of beer, or molasses in the case of the production of strong beverages, such as rum and similar, or still other products in function of the applications for which the reactor is intended.

The nutrient medium 5 concerned penetrates through the layer 16 into the layer 17 through which the yeasting process is started. As a result of this the nutrient medium is transformed into carbon dioxide gas that leaves the reactor via channel 2 and rises through the liquid in this channel, as well as alcohol. The gas and the alcohol can pass through the porous layer 16, but the organisms 3 which bring about the transformation, with the aforementioned bioconversion however always remain immobilised in the layer 17, at least as long as they have not died. Through the transformation of the nutrient medium in the layer 17 and the removal of the formed products a permanent shortage of these products develops with the result that through diffusion once again, also permanent, nutrient medium 5 is absorbed back into the layer 17.

Considering the gas formation and the formation of alcohols which are lighter than the nutrient medium 5, the overall density, measured per diameter in the channel 2, permanently decreases with the result that, because the density of the product 5 in the pipe 9 is always the greatest, through this difference in density an automatic flow in the circuit arises according to arrow A.

When the reactor is started small gas bubbles 23 first develop as shown in figure 4, through which the flow effect is relatively small. Once the reactor has been in operation for a time, such a great development of gas arises that, at least when the diameter of the channel 6 is chosen sufficiently small, gas bubbles 24 are formed which take up the whole diameter of the channel 6, all of which as shown in figure 5. Through the force exerted upwards by these rising gas bubbles 24 a relatively strong current develops in the circuit which promotes the reaction. In order to achieve this effect, each channel 6 preferably has a diameter of minimum 2 mm and maximum 10 mm. Very good results are achieved with a diameter of 2,5 mm. A too small diameter leads to the gas bubbles blocking, through which the aforementioned effect is lost.

The carbon dioxide gas gathers at the top in the reactor and is preferably removed and/or caught via an outlet 25. The alcohols formed by the yeasting process are lighter than the nutrient medium 5 and the possible residue from the reaction.

Through this some liquid 26 that principally exclusively consists of alcohols is also formed in the top of the reactor 1, while the nutrient medium 5 that has not yet reacted is taken along back through the feed back pipe 9 to the inlet 4 by the aforementioned circulation effect.

According to a first possibility the reactor is left in operation without further supply of the product 5 from the supply means 19 until the stream of liquid A has come or almost come to a standstill, after which the formed alcohol containing end product 27 is separated from the remaining products which have developed as a result of the reaction (bioconversion).

According to a second possibility the nutrient medium 5 can be permanently supplied in the pipe 9, from the supply means 19, while the formed alcohol containing product 27, thus the beer or strong beverage, to the degree that this is formed is separated by means of suitable separation means 28, for example consisting of a decantering device, and is removed via the outlet 29.

The temperature of the fermentation process can be controlled with a thermometer or any other temperature measurement device 30, whereby an adjustment of the temperature can be effected by means of a heat exchanger 31 through which a liquid at suitable temperature is directed.

The alcohol content can be measured at the outlet 29 by a measurement of density 32.

Notwithstanding that the present invention was described above on the basis of a laboratory arrangement, it is clear that it can be utilised in a similar manner and on a larger scale industrially.

In that case the porous structure 10 is equipped with several channels 6, all of which as shown in figure 6. The porous structure 10 is for example hereby formed by silicon carbide, analogue to the layer 17, while the aforementioned layer 16 of aluminium oxide is installed along the respective walls 7 of the channels 6. In figure 7 an industrial embodiment for the reactor according to figure 1 is again shown very schematically. In order to increase the productivity a large number of channels 6 are used. The catalyst or organisms are rinsed in for example from a reservoir 33 and by means of a pump 34.

The product 5, or thus the nutrient medium with a fermentation process, is let into the aforementioned circuit by means of a controlled valve 35, whereby the control of the valve 35 can be effected by means of a measurement of level 36 in the separation means 28.

The carbon dioxide gas can be caught in a tank 37 in order to be further applied in the production of beer.

The lighter formed fractions of the liquid, which contain the alcohols, are decanted and removed to a tank 40 via a pipe 38, provide with a controlled valve 39. The control of the valve 39, which is not shown in the figures, can for example be effected in function of a measurement of the alcohol content in the aforementioned uppermost part 26.

Finally it should be noted that the use of a porous structure 10, formed of materials such as aluminium oxide, silicon carbide, silicon oxide, boron carbide, aluminosilicate or materials of the type which provide for a similar structure, offers the advantage that the organisms 3 can grow permanently without the growth being hindered by dying organisms in this structure 10, such as is the case when semipermeable membranes are applied as described in the US 4.266.026 and the EP 112.812.

With the applications of a porous structure as meant in the present invention a process is always obtained whereby, as is hereafter clarified on the bases of figure 8, the permenant growth of the new organisms is possible. The organisms 3A which are found nearest to the channels 6, are better nourished than the organisms 3B which are found at greater distances from the channels 6. This has the result that the organisms 3A develop the quickest, but also die quicker, after which they disintegrate. The use of the aforementioned porous structure 10 allows, contrary to the use of a semipermeable membrane, that the formed remaining product of the disintegrated organism can be taken along with the liquid flow in the channels 6, with the result that the organism 3B can occupy the place of a disintegrated organism 3A, such that this organism 3B can develop in abundance, while in the place where the organism 3B was previously, the new growth of organisms can take place.

Notwithstanding the reactor 1 according to the invention is particularly suitable for realising a fermentation process it is clear that it can also be applied with non-biological conversions, thus chemical reactions between organic or inorganic chemical products. The reactor 1 need not principally provide for a closed circuit. Depending on the utilisation, for specific reactions it may be desirable that the products 5 only pass through the channel 6 once. According to another variant several reactor units are connected in cascade and in this way run through successively, whereby a feed back pipe 9 can be provided for or not from the last reactor unit to the first.

With the aforementioned fermentation process obviously other nutrient media and other organisms can also be applied such in function of the intended end product.

The present invention is in no way restricted to the embodiments described as examples and shown in the drawings, but such reactor, and the procedure for realising a fermentation process which such a reactor utilises, may be developed according to different variants without departing from the scope of the present invention.

## Claims

1.- Reactor, for the production of a bioconversion or chemical reaction of the type whereby a product to be treated is transformed by means of an auxiliary medium into a product of which the overall density is different from the density of the product to be treated, characterised in that the reactor principally consists of means (2) which can provide for the immobilisation of an auxiliary medium (3) dispersed in space, consisting of a porous structure (10); an inlet (4) for the supply of the product (5) to be treated; at least one free channel (6), that extends through the porous structure, extending upwards connected with one extremity to this inlet (4) and that allows that the product (5) to be treated makes contact along the walls (7) of this with the immobilised auxiliary medium (3); and an outlet (8) provided on the other extremity of the channel (6).

2.- Reactor according to claim 1, characterised in that the outlet (8) and the inlet (4) are connected to each other outside the core of the reactor (1) by means of a feed back pipe (9), such that this pipe (9) forms a circuit together with the channel (6) of the reactor (1).

3.- Reactor according to claim 2, characterised in that the whole circuit shows a free flow opening, such that this circuit permits the automatic circulation as a result of the appearing overall density difference.

4.- Reactor according to one of the preceding claims, characterised in that the channel (6), respectively the channels (6), are arranged standing perpendicular in the reactor (1).

5.- Reactor according to one of the preceding claims, characterised in that the porous structure (10) consists of one or several materials which allow the catalyst or similar to be rinsed in.

6.- Reactor according to claim 5, characterised in that the degree of fineness of the porosity of the structure (10) increases towards the channel (6), respectively the channels.

7.- Reactor according to claim 6, characterised in that the porous structure (10) principally consists of one layer (18) acting as a holder for the auxiliary medium (3) in which the fineness of the porosity gradually increases in the direction of the channel (6).

8.- Reactor according to claim 6, characterised in that the porous structure (10) principally consists of two layers fitting closely to each other as a physical entity, whereby the material of the layer (16) which borders on the aforementioned channel (6) shows the finest porosity, and whereby the layer (17) with the least fine porosity functions as holder for the auxiliary medium (3) and allows the penetration of specific particle shaped auxiliary media (3), while these are absolutely held back by the layer (16) with the finest porosity.

9.- Reactor according to claim 7 or 8, characterised in that the layer (17, 18) functioning as holder principally consists of silicon carbide.

10- Reactor according to claim 8, characterised in that the layer (16) with the finest porosity principally consists of aluminium oxide.

11.- Reactor according to one of the preceding claims, characterised in that the porous structure (10) on the outsides, more especially the sides which make no contact with the channel (6) of the reactor, are surrounded by a cover (11).

12.- Reactor according to claim 11, characterised in that a space (12) is present between the cover (11) and the porous structure (10), whereby the cover (11) has a supply opening (13) exiting herein.

13.- Reactor according to one of the preceding claims, characterised in that it has separating means (28) to separate the formed end products (27) from the product (5) to be treated which has not yet finished reacting.

14.- Reactor according to claim 13, characterised in that the separating means (28) principally consist of a decanting device placed on top of the reactor (1) which enables that lighter liquid fractions be separated.

15.-Reactor according to claims 2 through 14, in particular whereby use is made of a feed back pipe (9) as aforementioned, characterised in that supply means (19) for the product (5) to be treated are connected to the formed circuit, as well as means which control the amount of the product (5) supplied.

16.- Reactor according to claim 15, characterised in that the means which control the amount supplied principally consist of a pumping device (21).

17.- Reactor according to claim 15, characterised in that the supply means principally consist of a reservoir (20) that is placed higher than the reactor (1), while the means which control the amount of the product (5) supplied, principally consist of a controlled valve (35).

18.- Reactor according to one of the preceding claims, in particular for transformation whereby formation of gas appears, characterised in that the channel (6), respectively the channels (6) of the reactor (1), show a diameter which allows the gas bubbles (24) to occupy the whole cross section of the channels (6) concerned.

19.- Reactor according to one of the preceding claims, characterised in that the aforementioned channel (6), respectively channels (6), show a diameter of at least two millimetres.

20.- Procedure for realising a fermentation, characterised in that it principally consists in immobilising, around the channel (6) directed upwards, the organisms (3) which can be reached by the liquid in this channel (6), which bring about the fermentation in a porous structure which prevents the penetration of the living organisms into the aforementioned channel; the supply of a nutrient medium (5) for the fermentation process to the inlet (4) of the channel and the catching of the obtained products at the outlet (8) of the aforementioned channel (6).

21.- Procedure according to claim 20, characterised in that the product to be treated, thus the nutrient medium (5) is repeatedly transported via a free feed back pipe (9) through the actual reactor (1), whereby the formation of gas from the fermentation process is employed to provide the driving force necessary for the circulation.

22.- Procedure according to claim 20 or 21, characterised in that prior to the fermentation the organisms in the reactor (1) are immobilised by rinsing these into a porous structure (10) present herein which allows the absorption of the organisms (3), but absolutely prevents the penetration of these into the channel (6), after which the supply opening (13) along which the organisms are brought in, is closed off.

23.- Procedure according to claim 20, 21 or 22, characterised in that the gasses formed by the fermentation are removed at the top of the reactor.

24.- Procedure according to one of the claims 20 through 23, characterised in that the formed alcohol containing end product is withdrawn out of the top of the reactor (1) by decanting it.

25.- Procedure according to claim 22, characterised in that when the reactor (1) is loaded up with the product to be treated, this is left in operation without supply of further products until the stream of liquid has come or almost come to a standstill, after which the formed end products (27) are separated from the remaining products of the reaction.

26.- Procedure according to claim 21, characterised in that during the operation of the reactor nutrient medium (5) is permanently supplied to the circuit formed by the reactor (1) and the feed back pipe (9), while on the other hand at least the formed alcohol containing end product (27) is permanently drawn off.

27.- Procedure according to one of the claims 20 through 26, characterised in that the nutrient medium principally consists of wort.

28.- Procedure according to one of the claims 20 through 26, characterised in that the nutrient medium principally consists of a molasses environ-

ment.

*Fig.1*

Fig.2

Fig.3

Fig.8

Fig.4

Fig.6

Fig.5

Fig.7

EP 0 355 910 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 266 026 (B.R. BRESLAU)(05-05-1981) * Whole document * | 1-5,8, 11-28 | C 12 M 1/12 |
| Y | | 6,7,9, 10 | |
| Y | PATENT ABSTRACTS OF JAPAN, vol. 11, no. 18 (C-398)[2465], 17th January 1987, page 19 C 398; & JP-A-61 192 280 (TAKESHI KOBAYASHI) 26-08-1986 * Abstract * | 6,7,9, 10 | |
| X | EP-A-0 112 812 (MONSANTO CO.)(04-07-1984) * Page 4, line 25 - page 5, line 7; page 5, line 28 - page 9, line 20; examples 1-4; claims 1,2,4-7,9,10; figures 1-3 * | 1,4,5,8 ,11,12, 18,20, 22,23, 27,28 | |
| X | GB-A-2 159 729 (NORTON CO.)(11-12-1985) * Page 1, line 80 - page 2, line 104; page 3, lines 61-65,92-119; claims 1,12; figures 1-6 * | 1,4,5,8 -12 | |

TECHNICAL FIELDS SEARCHED (Int. Cl.5)

C 12 M

The present search report has been drawn up for all claims

| Place of search | Date of completion of the search | Examiner |
|---|---|---|
| THE HAGUE | 12-10-1989 | GROENENDIJK M.S.M. |

EPO FORM 1503 03.82 (P0401)